Europäisches Patentamt

European Patent Office

Office européen des brevets

(11) Publication number: **0 359 391**
**A1**

# EUROPEAN PATENT APPLICATION

(21) Application number: **89308087.9**

(51) Int. Cl.5: **A61F 13/15**

(22) Date of filing: **09.08.89**

| | |
|---|---|
| The title of the invention has been amended (Guidelines for Examination in the EPO, A-III, 7.3). | (71) Applicant: **W.R. Grace & Co.-Conn.**<br>**1114 Avenue of the Americas**<br>**New York New York 10036(US)** |
| (30) Priority: **12.08.88 US 231745** | (72) Inventor: **Schirmer, Henry George**<br>**156 Edgecombe Road** |
| (43) Date of publication of application:<br>**21.03.90 Bulletin 90/12** | **Spartanburg SC 29302(US)** |
| (84) Designated Contracting States:<br>**AT BE CH DE ES FR GB GR IT LI LU NL SE** | (74) Representative: **Senior, Alan Murray et al**<br>**J.A. KEMP & CO 14 South Square Gray's Inn**<br>**London WC1R 5EU(GB)** |

(54) **Hygienic absorbent pads.**

(57) The present invention provides an improved moisture absorbent pad. A moisture impermeable thermoplastic substrate (22,24) includes gas bubbles (28) with interstices between the bubbles for moisture absorbent material (32). A moisture permeable inner film (34) is adhered to portions of the substrate where the gas bubbles are formed.

FIG. 2

EP 0 359 391 A1

# ABSORBENT PADS

The present invention relates generally to absorbent pads, for example absorbent pads for use in articles such as disposable diapers and bed pads.

More particularly, the present invention relates to absorbent pads which are relatively non-squeezable, so as to avoid undesirable exudation of liquids absorbed in the pad.

Typical absorbent pads for use in, for example, disposable diapers, consist of first and second surface layers, at least one of which is a moisture permeable or moisture transmitting surface layer. An absorbent material such as ground wood pulp or other cellulosic materials is disposed in between these two surface layers. These abosrbent pads, disposable diapers, and the like typically work well until pressure is applied to the article. When this occurs, previously absorbed moisture can be squeezed out of the pad, thereby reducing its effectiveness as an absorbent article.

The present invention reduces this problem by providing a product which has absorbency but also resists squeezing out of previously absorbed contents.

Of interest is U.S. Patent No. 4,248,822 issued to Schmidt and disclosing a moisture permeable film covering for absorbent material.

According to the present invention there is provided a moisture absorbent pad including a moisture impermeable substrate with gas bubbles therein, moisture absorbent material between the bubbles and a moisture permeable film covering the moisture absorbent material and attached to the portions of the substrate where the bubbles are formed.

More particularly the present invention provides a moisture absorbent pad comprising a moisture impermeable thermoplastic substrate having a first, outer, film, a second film adhered to the first film, and gas bubbles entrapped between the first and second films; a moisture permeable inner film adhered to the portions of the second film of the substrate where the gas bubbles are formed; interstices between the gas bubbles in the space between the moisture permeable inner film and the second film of the substrate; and said interstices containing a moisture absorbent material.

In another aspect this invention provides a method of making a moisture absorbent pad comprising: providing a moisture impermeable thermoplastic film: shaping a second thermoplastic film; adhering the second film to the first film; and thereby forming gas bubbles between them; depositing moisture absorbent material in the interstices between the gas bubbles; and adhering a moisture permeable inner film to the portions of the second film of the substrate where the gas bubbles are formed.

The invention may be more readily understood by reference to the accompanying drawings, given by way of example and in which:

Figure 1 is a cross-sectional view of a typical prior art construction of an absorbent pad; and

Figure 2 is a cross-sectional view of an absorbent pad in accordance with the present invention.

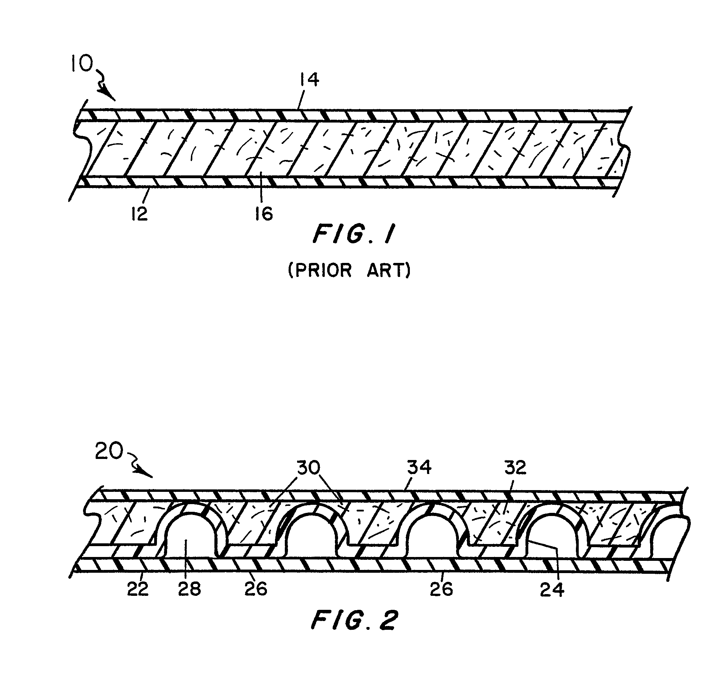

Referring to Figure 1, a typical absorbent pad 10 has a moisture impermeable layer 12 and a moisture permeable layer 14.

A layer of cellulosic fluff or other moisture absorbent material 16 is entrapped between the outer layers 12 and 14.

When absorbent pad 10 is compressed, the liquid or moisture previously absorbed into the material 16 can be squeezed out through permeable layer 14. This reduces the effectiveness of the absorbent pad 10 for its intended use.

To solve this problem, the present invention provides an improved absorbent pad 20.

Absorbent pad 20 includes a moisture impermeable thermoplastic substrate having a first outer film 22 and a second film 24, the second film adhered to first outer film 22 at segments 26 of the adjoining films.

Intermittently spaced between films 22 and 24 are gas bubbles 28.

The gas bubbles 28 are disposed so as to create interstices 30 between adjacent bubbles. Interstices 30 are filled with a moisture absorbent material 32 such as cellulosic fluff, or other moisture absorbers such as the hydrophilic synthetic polymers manufactured by W.R. Grace under the HYPOL trademark.

A moisture permeable inner film 34 is adhered to the second film 24 in areas where the gas bubbles occur in the pad. This moisture permeable film may be one with a moisture permeability sufficient for end uses such as those described above. It may also be a perforated film.

The present invention is particularly suitable for applications such as disposable diapers where the moisture may pass through permeable layer 34 and be absorbed into absorbent material 32. The gas bubbles 28 act as a structural support to prevent release of substantial amounts of absorbed liquids from material 32.

It is evident from the previous description that the total effective moisture absorbency of the absorbent pad 20 should be gauged not merely by the total amount of and type of moisture absorbent material 32 used as the filler within the pad, but also by the degree to which release of previously absorbed liquids is avoided.

Those skilled in the art will readily understand that the relative extent and pattern of distribution of gas bubbles 28 and interstices 30 can be modified in various ways to provide more or less moisture absorbent material 32 to the pad 20.

Various techniques may be used to produce the absorbent pad 20. In a preferred method, the first outer film is provided, and a second film adhered to the first film.

Before this adhering step, the second film has been heated, and shaped at intervals to form depressions in the film which form bubbles when closed by the first film. Moisture absorbent material may then be added to the interstices between the bubbles of the substrate, and a moisture permeable film thereafter added to complete the structure. The shaping of the second film may be by vacuumizing; it may be held by vacuum against a shaped plate and thus be shaped. A film adapted to include bubbles on heating or under vacuum may also be used.

## Claims

1. A moisture absorbent pad including a moisture impermeable substrate with gas bubbles therein, moisture absorbent material between the bubbles and a moisture permeable film covering the moisture absorbent material and attached to the portions of the substrate where the bubbles are formed.

2. A moisture absorbent pad comprising:

a) a moisture impermeable thermoplastic substrate comprising:
i) a first, outer, film,
ii) a second film adhered to the first film,
iii) gas bubbles entrapped between the first and second films;

b) A moisture permeable inner film adhered to the portions of the second film of the substrate where the gas bubbles are formed;

c) interstices between the gas bubbles in the space between the moisture permeable inner film and the second film of the substrate; and

d) said interstices containing a moisture absorbent material.

3. A moisture absorbent pad according to claim 1 or 2 wherein the gas bubbles are air bubbles.

4. A moisture absorbent pad according to claim 1, 2 or 3 wherein the moisture absorbent material includes at least one of a cellulosic material and a hydrophilic synthetic polymer.

5. A moisture absorbent pad according to claim 1, 2, 3 or 4 wherein the gas bubbles are distributed in a substantially regular pattern.

6. A moisture absorbent pad according to any preceding claim wherein the gas bubbles are between first and second films of the substrate.

7. A method of making a moisture absorbent pad comprising:

a) providing a moisture impermeable thermoplastic film;

b) shaping a second thermoplastic film;

c) adhering the second film to the first film; and thereby forming gas bubbles between them;

d) depositing moisture absorbent material in the interstices between the gas bubbles; and

e) adhering a moisture permeable inner film to the portions of the second film of the substrate where the gas bubbles are formed.

FIG. 1

(PRIOR ART)

FIG. 2

| | European Patent Office | EUROPEAN SEARCH REPORT | Application Number |
|---|---|---|---|

EP 89 30 8087

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 512 859 (OBERHUBER) <br> * Figure 2 * <br> --- | 1-7 | A 61 F 13/15 |
| X | DE-A-3 301 555 (VEREINIGTE PAPIERWERKE SCHICKEDANZ) <br> * Whole document * <br> --- | 1-7 | |
| X | GB-A-1 462 467 (A.S. RYAN) <br> * Whole document * <br> --- | 1-3,5-7 | |
| X | WO-A-8 201 991 (S.A. HYRY) <br> * Abstract; figures 2,3,7 * <br> --- | 1-3,5-7 | |
| X | FR-A-2 265 894 (THE PROCTER & GAMBLE CO.) <br> * Whole document * <br> ----- | 1-7 | |
| | | | TECHNICAL FIELDS SEARCHED (Int. Cl.5) <br><br> A 61 F |

The present search report has been drawn up for all claims

| Place of search | Date of completion of the search | Examiner |
|---|---|---|
| THE HAGUE | 11-10-1989 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)